# EUROPEAN PATENT APPLICATION

(11) **EP 1 314 358 A1**
(43) Date of publication of application: **28.05.2003**
(21) Application number: 01870252.2
(22) Date of filing: 21.11.2001
(51) Int. Cl.: A23K 1/16

(54) **Antimicrobial composition for animals**

(71) Applicant: N.V. Seghers Nutrition Sciences, 9031 Drongen (BE)
(72) Inventor: MOLLY, Koen, 9870 Olsene (BE); BRUGGEMAN, Geert, 8200 Brugge (BE)
(74) Representative: Brants, Johan Philippe Emile

(57) **Abstract**

The present invention relates to a composition comprising an MCFA component and at least one growth-promoting component selected from the group comprising organic acids, inorganic acids, animal feed antibiotics, conventional growth promoters, and plant extracts.

The invention further relates to the use of said composition as an antimicrobial agent and to an animal feed comprising said composition and to a method for the improvement of growth and/or for reducing feed conversion and/or for improving feed value and/or for improving health and well-being of an animal by providing said animal with a feed comprising a composition.

## Description

### Technical field

The present invention relates to an antimicrobial composition suitable as animal feed additive. A primary object of said antimicrobial composition is the improvement of the microbial ecosystem in the gastrointestinal tract. The present invention relates to an antimicrobial composition providing improved combined antibacterial and antifungal properties.

The present invention discloses further a process for improving the efficiency of feed intake and/or for promoting the growth of animals.

### Background of the invention

The well being and health and hence the economic value of livestock depends primarily on a good-functioning and well-balanced microbial ecosystem in the gastrointestinal tract of the host. Disturbance of this microbial eco-system will ultimately result in a decreased performance, often attended with infectious diarrhoea. An important source of said disturbance is the presence in the gastrointestinal tract of undesired micro-organisms as contaminating pathogens in the animal's feed. All food and feed ingredients are naturally contaminated with bacteria, yeast and fungi (mainly mould-forming), the latter usually in the form of spores. At temperatures above 4 °C, most foods and feeds are ideal media for microbial growth and most of the time for subsequent toxic metabolite (e.g. endotoxin, mycotoxin) production by the *in situ* development of micro-organisms.

Contamination of agricultural products with mycotoxin producing fungi is often unavoidable and of world-wide concern. Since humidity and temperature are important parameters for fungal growth, liquid food and feed are very susceptible to fungal contamination and growth and subsequent mycotoxin development. Mycotoxicosis can be caused by e.g. aflatoxins, ochratoxins, trichothecenes, zearalenones and citrinins. Mycotoxins cause a wide variety of adverse clinical signs depending on the nature and concentration of mycotoxins present, duration of exposure, the animal species, its age and nutritional health status at the time of exposure to the contaminated food and feed.

In view of the economical interest of modern animal husbandry systems to increase productivity and maintain profitability, it has become a general practice to increase the growth rate by subjecting specific animals such as piglets to an early weaning. This early weaning however burdens the animal with a lot of adverse stresses, mainly of nutritional origin and are often accompanied by a more or less severe decrease in feed intake and energy deficiency and thus leading to a mobilization of body reserves by the animal. Feed maldigestion and malabsorption may further aggravate the situation and result in digestive upsets mainly due to bacterial and fungal overgrowth in and/or viral infections of the gastrointestinal tract. Such digestive pathology problem leads to severe weight losses and an increased mortality amongst the animals.

The digestive pathology as described above also leads to a very inefficient feed conversion. Thus the feed taken up by the animal is not efficiently converted. A low FCR (Feed Conversion Ratio is the ratio of the amount of feed consumed relative to the weight gain of an animal) indicates that a given amount of feed results in a growing animal gaining proportionately more weight. The latter situation indicates that the animal is able to utilize the feed more efficiently.

The administration of traditional antimicrobials or feed antibiotics is one way in which the digestive pathology is prevented and hence a way to lower the FCR. Those skilled in the art appreciate, however, that antibiotic therapy for the treatment of numerous disorders and diseases results in the destruction of the intestinal microflora. This destruction of the intestinal microflora by the antibiotic results in the proliferation of pathological microorganisms. This can give rise to diseases like diarrhoea. Therefore, it has been necessary constantly to develop and use new antibiotics and mixtures thereof to the animals as described in e.g. EP Patent 597167 which discloses the use of an antibiotic mixture of gentamycin and lincomycin.

An important draw-back, however is that many antibiotics applied to the animals provoke resistance of the bacteria present in the animals and other microorganisms. Further, a number of the used antibiotics to the animals are the same or chemically very closely related to those antibiotics which are used in the fight against diseases provoked by bacteria and other micro-organisms in humans. As such, there has been an increasing concern in the public opinion that the resistance found in the microorganisms in the livestock will be transferred to the disease provoking microorganisms living in human beings, wherefore these cannot be controlled with the present human antibiotics at disposal.

Alternative feed additives to improve the animal's well-being and hence its ability to efficiently convert feed into food products are increasingly being used and include feed acidifiers for improved feed preservation and/or improved protection of the digestive tract against proliferation of pathogenic bacteria (WO 98/43634). Also medium-chain fatty acids (MCFA) having 6 to 12 carbon atoms have been reported for use in feedstock; e.g. US Patent no 5,462,967 discloses the use of MCFAs having 6 to 12 carbon atoms for anti-protozoiasis effect and suppression of an excess formation of systematic fat of, in particular, domestic fowl.

With regard to the relative expensive cost factor of feed in the production of food-producing animals, it is clear that an additional aspect of lowered feed conversions will directly improve the profitability of a feed producer.

It is an object of the present invention to provide a composition which provides an effective inhibition of digestive pathology in the animal's gastrointestinal tract. It is a further object of the present invention to provide a composition which improves the microbial balance of the intestinal microflora. It is a further object of the present invention to provide a composition which provides a low FCR without increasing the feed cost per unit weight. It is a further object to the present invention to provide a composition which enhances growth. It is a further object of the present invention to provide a method of enhancing weight gain and feed efficiency in an animal.

### Summary of the invention

The invention relates in general to a composition comprising a MCFA component and at least one additional growth promoting component selected from the group comprising organic acids, inorganic acids, animal feed antibiotics, conventional growth promoters and plant extracts like oreganum and allicin.

The compositions according to the present invention surprisingly led to a synergistic antimicrobial activity against bacteria, yeast, and moulds. Said compositions also lead to a much increased growth and a much reduced FCR.

The invention further relates to methods for improving growth and/or reducing feed conversion and/or for improving feed value and/or for improving health and well-being of an animal by providing said animal with a feed comprising a composition according to the present invention.

### Detailed description of the invention

Unless defined otherwise, all technical and scientific terms used herein have the same meaning as commonly understood to one of ordinary skill in the art.

In this specification and the appended claims, the singular forms "a", "an", and "the" include plural references unless dictated otherwise.

The present invention relates to improved feed conversion activities of animals without concomitant substantial or significant stimulation of food intake. Typically the food intake is the same or decreased in treated animals as compared to untreated animals. New compositions and methods for obtaining the above are provided.

As used herein the expression "improving the feed conversion" and cognate expressions such as "feed conversion improvement" refer to improving feed utilization efficiency and/or improving growth rate. That is, in accordance with the present invention, treated animals (as compared to untreated animals) can have substantially the same feed intake and grow at an increased growth rate, can have decreased feed intake and grow at substantially the same growth rate, or can have decreased feed intake and grow at an increased growth rate. The term "growth" as used herein refers to a gain in weight as in gained weight by increased tissue deposits on the animal's carcass yielding the choice meat.

In accordance with the present invention, in a first aspect, a composition is provided comprising a MCFA component and at least one growth-promoting component selected from the group comprising organic acids, inorganic acids, animal feed antibiotics, conventional growth promoters and plant extracts. In one embodiment, a composition is provided wherein the MCFA component consists essentially of MCFA, salts, or derivatives, or mixtures thereof. A particular suitable phase of the MCFA component according to the present invention is solid or semi-fluid, more in particular emulsions. Accordingly, the MCFA component of the composition according to the present invention may be a single MCFA, a single MCFA salt, a single MCFA derivative, a mixture of MCFAs, a mixture of MCFA salts, a mixture of MCFA derivatives, a mixture of MCFA's and MCFA salts, a mixture of MCFAs and MCFA derivatives, a mixture of MCFA salts and MCFA derivatives, a mixture of MCFAs, MCFA salts and MCFA derivatives.
As used herein, the term "MCFA component" refers to a fraction in the composition according to the present invention consisting essentially of MCFA, salts, or derivatives, or mixtures thereof. As used herein, the term "MCFA" refers to a medium chain fatty acid with said "medium chain fatty acid" meaning a saturated fatty acid, unsaturated fatty acid, or mixture thereof, having 6 to 14 carbon atoms. By "medium chain saturated fatty acid," as used herein, is meant C₆ (caproic), C₈ (caprylic), C₁₀ (capric), C₁₂ (lauric), or myristic (C₁₄) saturated fatty acids, or mixtures thereof. Particularly suitable is the use of a C₈/C₁₀ mixture in equal amounts. The C₇, C₉, C₁₁, and C₁₃ saturated fatty acids are not commonly found, but they are not excluded from the possible medium chain fatty acids.

As used herein, the term 'MCFA salt" refers to a salt of the free fatty acid. As used herein, the term "free fatty acid" refers to a, underivatised fatty acid, i.e. a fatty acid not converted into a salt, an amide, an ester etc. As used herein, the term MCFA derivative refers to a medium chain fatty acid whose carboxylic acid group is reversibly converted into another group to form amides, esters, glycerides. In this specification, the term MCFA derivative excludes MCFA salt.

As used herein, the term "growth-promoting component" refers to a compound or composition which exerts a growth-promoting activity or effect within a living vertebrate host, which excludes MCFA, salts, or derivatives thereof, or mixtures thereof. Said growth-promoting component is usually (part of) a feed additive or feed supplement.

Additional useful growth-promoting components include but are not limiting to prebiotics such as oligosaccharides including inulins, fructo-oligosaccharides, and galacto-oligosaccharides; probiotics such as *Bifidobacterium thermophilum* and *infantis, Lactobacillus acidophilus, bulgaricus, casei, lactis, plantarum,* and *reuteri, Bacillus subtilis* and *cereus, Streptococcus faecium, diacetilactus,* and *thermophilus, Enterococcus faecium* and *faeclum, Torulopsia, Aspergillus oryzae,* and *Streptomyces;* including their vegetative spores and synthetic derivatives; synbiotics; enzymes such as acid proteases from *Rhizopus* *rhizopodiformis, Aspergillus niger,* and the genus *Tramates,* and neutral to alkaline proteases from *Bacillus subtilis, licheniformis,* and *natta;* herbs such as *Mimosaceae* and *oregano,* including extracts and essential oils thereof; and fungi belonging to the class *Basidiomycotina* comprising genera belonging to the orders *Agaricales* and *Aphyllophorales,* such as *Armillariella mellea* (Fr.) Karst, *Tricholoma matsutake* (S. Ito et Imai) Sing., *Lentinus edodes* (Berk.) Sing. and Shiitake, *Coriolus versicolor* (Fr.) Quel., *Grifola gigantea* (Fr.) Pilat, Favolus Arcularius (Fr.) Ames, Ganoderma (Reishi), Cordyceps, Coriolus or Grifola (Maitake), either alone or in any combination with each other may be used as growth-promoting components in this specification. Other bio-active components such as present in blood plasma are also suitable. Suitable species are further: Lawsonia sp. en Serpulina sp.

The presence of a MCFA component in combination with a growth-promoting component in said new composition, according to the present invention has a synergistic effect on feed conversion improvement leading to much reduced FCRs.

Compared to FCR's obtained with single-growth-component feed additives, compositions of the present invention provides substantially higher feed conversion and substantially lower FCR. As used herein, the term "single-growth-component feed additive" refers to an additive comprising at least one growth component within a single class of growth-promoting components. The term "class" of growth-promoting components as used herein refers to a group or set of growth components sharing common characteristics and/or functionalities such as e.g. class of antibiotics, class of prebiotics, class of probiotics, class of synbiotics, class of herbs, class of conventional growth promoters, class of organic acids, and class of inorganic acids.

Compositions of the present invention provides up to 4-times higher antimicrobial effects compared to results obtained with single-growth-component feed additives.

In one embodiment, the MCFA in the composition according to the present invention is selected from the group of medium chain saturated fatty acids. Useful medium chain saturated fatty acids include but are not limited both even and odd fatty acids such as caproic acid (C₆), heptanoic acid (C₇), caprylic acid (C₈), pelargonic acid (C₉), and capric acid (C₁₀), including mixtures of each other. In the present invention, particular useful are mixtures of specific different saturated fatty acids, the individual specific saturated fatty acids containing a different number of carbon atoms. Such mixtures provide optimal antimicrobial properties.

In homologues series of fatty acids, the bactericidal efficiency has been found to increase with increasing chain length. Fatty acids with a chain length of 8 to 10 carbon atoms show optimal antimicrobial activity.

In the present specification, undecanoic acid and lauric acid are suitable medium chain saturated fatty acids and also butyric acid, valeric acid, tridecanoic, and myristic acid may be equally suitable in the present invention.

Substituted MCFAs such as 2-methyl-tetradecanoic acid, 5-methyltetradecanoic acid, 2,2-dimethyltetradecanoic acid, including, branched, monobasic, and dibasic fatty acids may be equally suitable in the present invention. In particular the non-synthetic acids are suitable.

In a particular useful embodiment, the MCFA in the composition according to the present invention is selected from the group of C₈₋₁₀ medium chain saturated fatty acids.

The currently accepted theory for the mechanism according to which the fatty acids exert antimicrobial activity is that the lipid microbial cell membrane is permeable for the undissociated fatty acid, as a consequence of which the fatty acid is capable of passing across the microbial cell membrane towards the more alkaline interior. Because of the higher intracellular alkalinity, the fatty acid is dissociated, thus involving a decrease of the intracellular pH below the survival level. The fatty acid thus in fact acts as a protonophore, which increases inward leak of H⁺ and which makes that the efflux of H⁺ is too slow to allow the intracellular pH to be increased again. The physicochemical properties of the fatty acids which allow them to act as protonophores, may vary and depend on numerous parameters. Examples of such parameters are the chain length and pKa of the fatty acid as well as the physicochemical environment, precipitations, the pH at the place of action and the chemical composition of the microbial envelope which determines the passage of the fatty acids through the membrane. In this respect, the better performance of the fatty acid containing 8-10 carbon atoms is attributed to the extreme permeability of the microbial cell membrane for this fatty acid.

Concentrations up to 1x10⁵ ppm of MCFA component in the animal feed are suitable. In further embodiment, up to 5x10⁴ ppm, in a further embodiment up to 1x10⁴ ppm, in a further embodiment up to 5x10³ ppm, and yet in a further embodiment up to 1.5x10³ ppm in the animal feed are useful concentrations. In a further embodiment concentrations as low as 10² ppm were effective.

According to a further embodiment, a composition is provided wherein the MCFA component comprises at least one MCFA salt and at least one MCFA derivative.

According to a further embodiment, a composition is provided wherein the MCFA component comprises at least one MCFA salt. In this specification, mixtures of two or more salts of the free fatty acids are particulary suitable. Non-limiting examples of suitable MCFA salts in the present invention include alkali metal salts such as lithium salts, sodium salts, potassium salts, and the like; alkaline earth metal salts such as magnesium salts, calcium salts, barium salts, and the like; various metal salts such as zinc salts, aluminum salts, iron salts, manganese salts, and the like; organoamine salts such as monoethanloamine salts, diethanoamine salts, trietholamine salts, and the like; basic amino salts such as lysine salts, ornithine salts, arginine salts, histidine salts, hydroxylysine salts, and the like; and alkali metal salts such as ammonium salts, and basic amino acid salts.

In a further embodiment of the present invention, a composition is provided wherein the MCFA comprises at least one MCFA salt selected from the group comprising ammonium salts, sodium salts, potassium salts, and calcium salts.

The use amount of MCFA salts in the present invention in animal feed is usually 0.001% to 25% by weight. The use amount of MCFA salts varies with factors such as the animal host, the animal's age, the kind of ingredients other than MCFA salts etc.

According to a further embodiment, a composition is provided wherein the MCFA component is at least one MCFA derivative. Suitable MCFA derivatives are selected from the group comprising mono-, di-, and tri-glycerides. A molecule of mono-, di-, or triglyceride is composed of a backbone of glycerol to which respectively one ("mono"), two ("di") or three fatty acids ("tri") are bound. Any combination of saturated, monounsaturated, or polyunsaturated fatty acids can be in a mono-, di-, or triglyceride molecule. A monounsaturated fatty acid has one site where hydrogen atoms can be added. A polyunsaturated fatty acid has two or more sites for additional hydrogen atoms.

Examples of naturally ocurring substances which are rich in MCFA derivatives such as mono, di, and triglycerides include but are not limited to coconut oil, palm kernel oil, babassu oil, cohune oil, tacum oil, cuphea oil derived from plant seeds, milk of mammalian species, such as milk from horse, rat, goat and rabbit, or butterfat. Also commercial sources of chemically structured or tailor-made mono-, di-, and triglycerides may be useful.

Suitable concentrations of MCFA derivatives such as mono-, di-, and triglycerides in the feed are ranged from 0.20% to 12% by weight. In a further embodiment, MCFA derivative concentrations may range from 0.25% to 10% by weight in the feed. In a further embodiment, MCFA derivative concentrations may range from 0.5% to 5% by weight in the feed.

In one embodiment of the present invention, the growth promoter component may be a single compound or a mixture of compounds within a single class of growth-promoting components or mixtures of single or two or more compounds from a single or two or more classes.

In one embodiment of the present invention, a composition is provided wherein the growth-promoting component is at least one component selected from the group comprising organic acids and inorganic acids. Accordingly, the growth-promoting component of the composition according to the present invention may be a single organic acid, a single inorganic acid, a mixture of organic acids, a mixture of inorganic acids or a mixture of organic and inorganic acids.

In a further embodiment, a composition is provided wherein the growth-promoting component is an organic acid. In a further embodiment, a composition is provided wherein the growth-promoting component is an organic acid being a C₁₋₈ carboxylic acid selected from the group comprising unsubstituted carboxylic acids such as formic acid, acetic acid, propionic acid, butyric acid, valerianic acid, and caproic acid, substituted carboxylic acids such as adipic acid, maleic acid, succinic acid, citric acid, fumaric acid, tartaric acid, lactic acid, gluconic acid, malic acid, and ascorbic acid, including cyclic carboxylic acids such as picolinic acid. The organic acid component may be a single unsubstituted carboxylic acid, a single substituted carboxylic acid, a mixture of unsubstituted carboxylic acids, a mixture of substituted carboxylic acids and a mixture of unsubstituted carboxylic acids and substituted carboxylic acids including saturated, unsaturated, cyclic and aliphatic carboxylic acids and metal complexes and salts thereof. Also single racemic forms and racemic mixtures may be used.

In a further embodiment, when added in feed for animals, useful concentrations of such carboxylic acids range from 0.05 eq/kg to 0.25 eq/kg feed. In further embodiments, concentrations of such carboxylic acids range from 0,10 eq/kg to 0.20 eq/kg feed, from 0,10 eq/kg to 0.15 eq/kg feed or from 0.12 eq/kg to 0.18 eq/kg feed.

In yet a further embodiment, a composition is provided wherein the growth-promoting component is an inorganic acid including strong inorganic acids in small quantities such as perchloric acid, hydroiodic acid, hydrobromic acid, hydrochloric acid, sulfuric acid, and nitric acid and weak inorganic acids such as phosphoric acid, hydrofluoric acid, hypochlorous acid, and nitrous acid.

Suitable concentrations of weak and strong inorganic acids which are useful range from 0.1-0.3% to 1-1.5% by weight.

In another embodiment according to the present invention, a composition is provided wherein the growth-promoting component is an animal feed antibiotic. Non-limiting examples of useful animal feed antibiotics include avoparcin, bacitracin such as zinc bacitracin, chlortetracyclin, flavophospholipol (flavomycin), furadox, gentamicin, lasalocid, lincomycin, monensin, neomycin, oleandomycin, oxytetracyclin, sarimomoicin, spiramycin, sulfametazine, tetracycline, tylosin, virginiamycin. In a further embodiment, a useful concentration of an animal feed antibiotic ranges from 0.5 ppm to 250 ppm in animal feed. In further embodiment, a useful antibiotic concentration for animal feed ranges from 1 ppm to 150 ppm. In further embodiment, a useful antibiotic concentration for animal feed ranges from 2 ppm to 100 ppm. In further embodiment, a useful antibiotic concentration for animal feed ranges from 2.5 ppm to 50 ppm. In yet a further embodiment, a useful antibiotic concentration for animal feed ranges from 2.5 ppm to 5 ppm.

In another embodiment according to the present invention, a composition is provided wherein the growth-promoting component is a conventional growth promoter selected from the group comprising nitrovin, olaquindox, carbadox, and cyadox. Concentrations of such conventional growth promoters ranges from 20 ppm to 150 ppm but usually range from 50 ppm to 100 ppm.

In a further embodiment, a composition according to the present invention comprises an amount from 50% to 99.9% by weight of MCFA component. In a further embodiment, an amount from 70% to 99.9% by weight of MCFA component is comprised in a composition according to the present invention. In a further embodiment, an amount from 85% to 99.9% by weight of MCFA component is comprised in a composition according to the present invention. In yet a further embodiment, an amount from 95% to 99.9% by weight of MCFA component is comprised in a composition according to the present invention.

According to this specification, a composition is provided comprising a MCFA component and at least one growth-promoting component. It is generally recognized by a skilled person in the art that supplemental ingredients are being used, e.g. various additives such as amino acids, minerals, vitamins, enzymes, etc., with various objects, such as nutrient replenishment, nutrient fortification, improvements of digestion and absorption, disease prevention, etc.

In a further embodiment, a composition according to the present invention is particularly useful as a microbial agent, particularly in livestock feed. In a further embodiment, a composition according to the present invention is particularly useful for animal feed. In a further embodiment, a composition according to the present invention is particularly useful for poultry and pig feed.

Accordingly, the use of a composition according to present invention has been proven to be particularly suitable for intestinal ecosystem improvement and/or lowering feed conversion ratio's and/or growth enhancement.
Further, a composition of the present invention may be suitable for intestinal function improvement and/or carcass quality improvement. When fed to dairy animals such as dairy cows, goats and ewes, a composition of the present invention may improve milk production.

It is a second aspect of the present invention to provide an animal feed comprising the synergetic composition according to the invention from 0.001% to 20% by weight and comprising standard animal feed from 80% to 99.999% by weight. As used herein, the term "standard animal feed" refers to feed for meat-producing animals, i.e. feed that can be used in the animal husbandry field and is suitable to be fed to meat-producing animals to supply part or all of the meat-producing animal's nutrient requirements. Main ingredients of standard animal feed include but are not limited to wheat flour, starch, dextrin, cereal grains such as corn, milo, etc., oil seed meals, such as soybean meal, rapeseed meal, cottonseed meal, linseed meal, chaffs and brans, such as rice bran, de-oiled rice bran, wheat bran, fish meals, oils and fats, such as beef tallow, soybean oil, palm oil, coconut oil, and fish oil.

In a further embodiment, said animal feed comprises a composition according to the present invention from 0.01% to 10% by weight. In a further embodiment, said animal feed comprises a composition according to the present invention from 0.1% to 10% by weight. In a further embodiment, said animal feed comprises a composition according to the present invention from 0.5% to 5% by weight. In a further embodiment, said animal feed comprises a composition according to the present invention from 0.1% to 5% by weight. In a further embodiment, said animal feed comprises a composition according to the present invention from 0.5% to 2% by weight.

According to the present invention, an animal feed is provided for use as feed for meat-producing animals. As herein used, the term "meat-producing animals refer to livestock animals including but not limited to cattle such as ruminants, sheep, swine including pigs and hogs, horses, and poultry, and their progeny, such as sucking pigs, piglets, calves, lambs kids, foals and chickens etc.
Also fishes, such as eel, carps, trout, rainbow trout, yellowtail, sea bream, silver salmon, gold fishes, colored carp, tropical fishes; shellfishes; crustaceans; are examples of the animals covered by the composition and/or animal feed of the present invention.
Also pets, such as dogs, cats, rabbits, hamsters, and their progeny are examples of the animals covered by the composition and/or animal feed of the present invention.

In a further embodiment, an animal feed according to the present invention is used for poultry or pig feed, in particular during the early life stages. Non-limiting examples of poultry that may be considered in the present invention include chickens, ducks, geese, guinea fowl, peafowl, pigeons, and turkeys. As used herein, the wording "during early life stage of poultry" refers to a life stage comprised between 1 day and 4 weeks. As used herein, the wording "during early life stage of pig" refers to a life stage comprised between 1 week before and 1 week, preferably 2 weeks after weaning. In a preferred embodiment an animal feed according to the invention is fed to piglets during a period comprised between weaning and up to 20 kg. In a further preferred embodiment an animal feed according to the invention is fed to poultry during a period comprised between 1 day and 4 weeks.

A further aspect of the present invention relates to a method for the improvement or prevention of digestive pathology and/or growth improvement and/or for reducing feed conversion and/or for improving feed value and/or for improving health and well-being of an animal by providing said animal with a feed comprising a composition as provided in this specification.

The compositions of the present invention may be added either directly to the animal's feed or may be added as a premix. The compositions according to the present invention will mostly be present in a form of a dry powder or mini-granules but may also be present in humidified, pasta-like emulsion-like or liquid form. A powder additive may be prepared by dryblending of the ingredients, but it may also be prepared by wet-blending of the ingredients followed by drying, grinding and possible sifting. A mini-granulate may be prepared the same way although to begin with, a sufficient amount of liquid may be added of an appropriate kind (e.g. water) in order to obtain sufficient adhesion power between the particles of the ingredients.

Accordingly, the present invention relates to induction by feeding the animal with a composition of the present invention of changes in the microbial ecosystem in the gastrointestinal tract of the animal in a specific way resulting in an improved gastrointestinal ecosystem. In particular when using *Lentinus edodes,* the total amount of enteric pathogens are in a first stage enumerated in the gastrointestinal tract, i.e. a selective development of the enteric pathogens within the gastrointestinal tract, and in a second stage, the enumerated enteric pathogens are very quickly excreted from the gastrointestinal tract of the animal. As used herein, the term "enteric pathogens" refers to an undesired population of pathogens.

The compositions according to the present invention have antimicrobial, especially antifungal and/or antiviral and/or antibacterial activity.

As will be appreciated, there are a large number of such pathogens including but not limiting to Gram negative bacteria like *Salmonella, Escherichia, Shigella, Klebsiella, Erwinia, Yersinia, Campylobacter, Helicobacter, Vibrio,* and *Pseudomonas;* Gram positive bacteria like *Clostridium;* viruses like Norwalk virus, Norwalk-like viruses and Rotavirus; protozoa like *Crytosporidium, Entamoeba, Giardia,* and *Dientamoeba;* fungi from the genera comprising *Aspergillus, Candida, Cephalosporum, Fusarium, Penicillum* including fungi belonging to the Fungi Imperfecti; yeast including *Saccharomyces* and *Hemiascomycetes.* The present invention would be suitable for the suppression of the development these pathogens in feed and in the gastrointestinal tract of animals.

By fast elimination of enteric pathogens from the gastrointestinal tract, in a second instance, better performances, which reflect in daily growth and feed conversion of the treated animals are obtained. The overall growth promoting affect of the feed additive composition of the present invention is readily and clearly visible at the elution level of the enriched enteric pathogen population from the gastrointestinal tract of the animal.

The administration of a composition according to the present invention to animals allows for a specific sequential action in the gastriontestinal tract, i.e. the enumeration of the enteric pathogens prior to a wash-out of the enumerated enteric pathogens. As a result of the synergistic action of the compositions of the present invention, efficient inhibition of microbial outgrowth by killing of the microbial cells is obtained.

### Examples

The following examples of the invention are exemplary and should not be taken as in any way limiting.

### Example 1: Antimicrobial effect of the compositions of the present invention on bacteria, yeast, and mould development.

As used herein, the term "control feed" refers to a standard animal feed for meat-producing animals.

Solid feed was used. The test was performed *in vitro.* No tests *in vivo* were performed.

The antimicrobial effect on development of bacteria, yeast and moulds was monitored (Table 1.)

Evaluation of antimicrobial effect was performed as follows:

Microorganisms were incubated with test substance (eventually blank) at different concentrations. This way, no growth was observed at particular concentration (Minimal Inhibition Concentration). This concentration was compared with antimicrobial activity obtained with control (blank) solution. Relative antimicrobial activity is expressed in Table 1.

**Table 1.**

| The indications "+" indicate relative antimicrobial values. Values "++(++)" or "++(+)+" or "+++(+)" indicate small quantitative differences compared with "++++". | | | |
|---|---|---|---|
| Diet | Antimicrobial effect | | |
| | Bacteria | Yeast | Moulds |
| control | = | = | = |
| control + *Lentinus edodes* | *++* | *++* | *++* |
| control + formic acid | + | + | + |
| control + phophorous acid | + | + | + |
| control + Salinomycine | + | + | + |
| control + Amoxyciline | + | | |
| control + inulin | + | + | + |
| control + Bacillus sp. | + | + | + |
| control + Oregostim | + | + | + |
| control + MCFA's | ++ | ++ | ++ |
| control + *Lentinus edodes* + MCFA's | ++++ | ++++ | ++++ |
| control + formic acid + + MCFA's | ++++ | ++++ | ++++ |
| control + phophorous acid + MCFA's | ++++ | ++++ | ++++ |
| control + Salinomycine + MCFA's | +++(+) | +++(+) | +++(+) |
| control + Amoxyciline + MCFA's | ++++ | | |
| control + inulin + MCFA's | ++(+)+ | ++(+)+ | ++(+)+ |
| control + Bacillus sp. + MCFA's | ++(+)+ | ++(+)+ | ++(+)+ |
| control + Oregostim + MCFA's | ++(++) | ++(++) | ++(++) |

From Table 1, it is clear that the combination of the use of a MCFA component with a growth-promoting component as in the compositions of the present invention has a synergistic effect on antimicrobial activity. We conclude that compared to antimicrobial activity obtained with single-growth-component feed additives, feed additives comprising a composition according to the present invention provides up to 4 to 3 times higher antimicrobial effects.

For the *in vivo* poultry trials, following set up was performed:

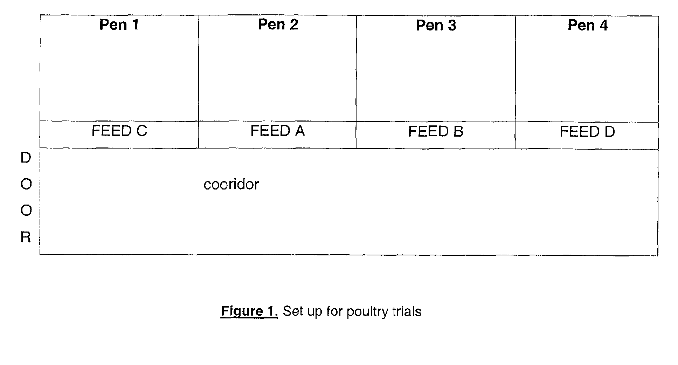

One battery was consisted of 4 pens (see Figure 1). The animals were one-day chickens with a density of 40 animals per pen. the animals were kept on straw and the total area per pen was 2 m². At the start of the trial, the chikkens were warmed by an infra red bulb and lightned constantly. The chickens were fed different feed *at libitum,* as illustrated in table 2. Feed A was control feed, feed B was control feed + MCFAs, feed C was control feed + MCFAs + additive, feed D was repitition of feed C. At regular time intervals, the chickens were weighed individually and feed intake was measured. Next, feed conversion was calculated. The results are illustrated in table 2.

**Table 2.**

| Influence of MCFA's in combination with different agents on chicken performance | | |
|---|---|---|
| Diet | Daily growth | FCR |
| control | = | = |
| control + *Lentinus edodes* | *++* | *--* |
| control + formic acid | + | - |
| control + phophorous acid | + | - |
| control + Salinomycine | + | - |
| control + Amoxyciline | + | - |
| control + inulin | + | - |
| control + Bacillus sp. | + | - |
| control + Oregostim | + | - |
| control + MCFA's | ++ | -- |
| control + *Lentinus edodes +* MCFA's | ++++ | ---- |
| control + formic acid + + MCFA's | ++(++) | --(--) |
| control + phophorous acid + MCFA's | ++(++) | --(--) |
| control + Salinomycine + MCFA's | ++(++) | --(--) |
| control + Amoxyciline + MCFA's | ++(++) | --(--) |
| control + inulin + MCFA's | +++(+) | ---(-) |
| control + Bacillus sp. + MCFA's | +++(+) | ---(-) |
| control + Oregostim + MCFA's | +++(+) | ---(-) |

From Table 2, it is clear that the combination of the use of a MCFA component with a growth-promoting component as provided by the compositions of the present invention has a synergistic effect on growth and feed conversion ratio of the animal. We conclude that compared to FCR's obtained with single-growth-component feed additives, the compositions of the present invention provides substantially higher feed conversion and substantially lower FCRs.

## Claims

1. A composition comprising an MCFA component and at least one growth-promoting component selected from the group comprising organic acids, inorganic acids, animal feed antibiotics, conventional growth promoters, and plant extracts.

2. A composition according to claim 1, wherein the MCFA component consists essentially of MCFA, salts, or derivatives, or mixtures thereof.

3. A composition according to claim 2, wherein the MCFA is selected from the group comprising caproic acid (C₆), heptanoic acid (C₇), caprylic acid (C₈), pelargonic acid (C₉), and capric acid (C₁₀), including mixtures of each other.

4. A composition according to claim 3, wherein the MCFA is selected from the group of medium chain saturated fatty acids comprising caprylic acid (C₈), pelargonic acid (C₉), and capric acid (C₁₀), including mixtures of each other.

5. A composition according to any previous claims 1-2, wherein the MCFA component comprises at least one MCFA salt and at least one MCFA derivative.

6. A composition according to any previous claims 1, 2, and 5, wherein the MCFA component comprises at least one MCFA salt.

7. A composition according to any previous claims 1, 2, 5, 6, wherein the MCFA salt is selected from the group comprising ammonium salts, sodium salts, potassium salts, and calcium salts.

8. A composition according to any previous claims 1, 2, 5, wherein the MCFA component is at least one MCFA derivative.

9. A composition according to any previous claims 2, 5, 8, wherein the MCFA derivatives are selected from the group comprising mono-, di-, and tri-glycerides.

10. A composition according to any of previous claims 1-9, wherein the growth-promoting component is selected from the group comprising organic acids and inorganic acids.

11. A composition according to claim 10, wherein the organic acid is a carboxylic acid selected from the group comprising C₁₋₈ substituted, unsubstituted, saturated, and unsaturated carboxylic acids.

12. A composition according to any of previous claims 1-11, wherein the growth-promoting component is an animal feed antibiotic.

13. A composition according to any of previous claims 1-12, wherein the growth-promoting component is a conventional growth promoter selected from the group comprising nitrovin, olaquindox, carbadox, and cyadox.

14. A composition according to any of previous claims 1-13 comprising a MCFA component in an amount from 50% to 99.9% by weight.

15. Use of a composition according to any of the previous claims 1-14 as an antimicrobial agent.

16. Use of a composition according to claim 15 for livestock feed.

17. Use of a composition according to any of the previous claims 1-14 for intestinal pathology improvement or prevention.

18. Use of a composition according to claim 1-14 for lowering feed conversion ratio.

19. Use of a composition according to any previous claims 1-14 for poultry or pig feed, in particular during the early life stages.

20. An animal feed comprising a composition according to any of previous claims 1-14 in an amount from 0.001% to 20% by weight.

21. A method for the improvement of growth and/or for reducing feed conversion and/or for improving feed value and/or for improving health and well-being of an animal by providing said animal with a feed comprising a composition according to any of the previous claims 1-14.
